# EUROPEAN PATENT SPECIFICATION

(11) **EP 1 712 225 B1**
(45) Date of publication and mention of the grant of the patent: **06.04.2011**
(21) Application number: 06015782.3
(22) Date of filing: 29.01.2002
(51) Int. Cl.: A61K 31/00, A61K 31/496, A61K 45/06, A61P 25/24, A61P 25/18, A61P 25/28, A61P 25/16, A61P 25/06, A61P 25/30, A61P 15/00, A61P 3/04, A61P 1/08

(54) **Substituted carbostyril derivatives as 5-HT 1A receptor subtype agonists for the treatment of bipolar disorder**
Substituierte Carbostyrilderivate als 5-HT1A-Subtyp Agonisten zur Behandlung von bipolarer Störung
Dérivés substitués de carbostyril comme agonistes du sous-type du récepteur 5-HT1A pour le traitement de troubles bipolaires

(30) Priority: 29.01.2001 US 770210
(43) Date of publication of application: 18.10.2006
(62) Divisional of application: 05023971.4
(73) Proprietor: OTSUKA PHARMACEUTICAL CO., LTD., Chiyoda-ku Tokyo 101-8535 (JP)
(72) Inventor: Uwahodo, Yasufumi, Tokushima-shi, Tokushima 771-1151 (JP); Jordan, Shaun, Frederic, Maryland, MD 21704 (US); Kikuchi, Tetsuro, Tokushima-shi, Tokushima 771-0104 (JP); Tottori, Katsura, Tokushima 771-1345 (JP); Hirose, Tsuyoshi, Tokushima-shi, Tokushima 770-0021 (JP)
(74) Representative: HOFFMANN EITLE

(56) References cited:
- EP-A- 0 367 141
- WO-A-99/38864
- WO-A-02/102297
- WO-A-03/026659
- US-A- 4 734 416
- US-A- 5 691 330
- LIEBERMAN JEFFREY A: "Atypical antipsychotic drugs as a first-line treatment of schizophrenia: A rationale and hypothesis." JOURNAL OF CLINICAL PSYCHIATRY, vol. 57, no. SUPPL. 11, 1996, pages 68-71, XP008005973 ISSN: 0160-6689
- MELTZER H Y ET AL: "SINGLE OR MULTIPLE RECEPTOR TARGETS: WHICH ARE BEST FOR ANTIPSYCHOTIC DRUGS" NEUROPSYCHOPHARMACOLOGY, ELSEVIER SCIENCE PUBLISHING, NEW YORK, NY, US, vol. 23, no. S02, August 2000 (2000-08), page S73, XP008029267 ISSN: 0893-133X
- PRINSSEN ERIC P M ET AL: "Interactions between neuroleptics and 5-HT1A ligands in preclinical behavioral models for antipsychotic and extrapyramidal effects." PSYCHOPHARMACOLOGY, vol. 144, no. 1, May 1999 (1999-05), pages 20-29, XP002209298 ISSN: 0033-3158
- UWAHODO YASUFUMI ET AL: "Pharmacological profile of OPC-14597, a novel antipsychotic drug (2): Weak extrapyramidal side effects." JAPANESE JOURNAL OF PHARMACOLOGY, vol. 67, no. SUPPL. 1, 1995, page 144P, XP001237224 68th Annual Meeting of the Japanese Pharmacological Society;Nagoya, Japan; March 25-28, 1995 ISSN: 0021-5198
- GARCIA-NANYA M., APIQUIAN R., FRESAN A.: "Atypical antipsychotics: Review article [Los antipsycoticos atipicos: Una revision]" SALUD MENTAL, vol. 24, no. 5, October 2001 (2001-10), pages 37-43, XP008005976
- KECK P E ET AL: "BIPOLAR DISORDER" MEDICAL CLINICS OF NORTH AMERICA, W. B. SAUNDERS COMPANY, PHILADELPHIA, US, vol. 85, no. 3, May 2001 (2001-05), pages 645-661, XP008005975 ISSN: 0025-7125
- JORDAN S ET AL: "IN VIVO EFFECTS OF ARIPIPRAZOLE ON DOPAMINERGIC AND SEROTONERGIC FUNCTION IN RAT PREFRONTAL CORTEX AND STRIATUM" SOCIETY FOR NEUROSCIENCE ABSTRACTS, SOCIETY FOR NEUROSCIENCE, US, vol. 2, no. 27, 2001, page 2327,AN87503, XP008005982 ISSN: 0190-5295
- GOODNICK, PAUL J. ET AL: "Aripiprazole: profile on efficacy and safety" EXPERT OPINION ON PHARMACOTHERAPY , 3(12), 1773-1781 CODEN: EOPHF7; ISSN: 1465-6566, 2002, XP008068613
- MCELROY S L ET AL: "Pharmacologic agents for the treatment of acute bipolar mania." BIOLOGICAL PSYCHIATRY 15 SEP 2000, vol. 48, no. 6, 15 September 2000 (2000-09-15), pages 539-557, ISSN: 0006-3223

## Description

### BACKGROUND OF THE INVENTION

### FIELD OF THE INVENTION

The present invention relates to a compound for use in treating a patient suffering from a disorder of the central nervous system associated with the 5-HT_{1A} receptor subtype. The active ingredient comprise a carbostyril derivative or a salt thereof.

### RELATED ART

U.S. Patent No. 5,006,528; European Patent No. 367,141 and Japanese Patent Kokai (Laid-open)7-304,740 (1995) contain the same chemical structural formula as the carbostyril derivatives in the present invention, and their pharmacological properties are beneficial drug treatments for schizophrenia.

Carbostyril compounds, as well as those disclosed in Japanese Patent Kokai (Laid-open)9-301,867 (1997) are useful for the treatment of anxiety.

The carbostyril derivatives disclosed in European Patent No. 226,441 have the genus of the carbostyril derivatives in the present invention, and they are useful for the treatment of hypoxia.

In addition to the above, the carbostyril derivatives disclosed in U.S. Patent No. 4,734,416; Canadian Patent No. 1,117,110; British Patent No. 2,017,701; German Patent Nos. 2,911,108, 1,912,105 and 2,953,723; Japanese Patent Kokai(Laid-open)Nos. 54-130,587 (1979), 55-127,371 (1980) and 62-149,664 (1987) have the genus of the carbostyril derivatives in the present invention, and they have antihistaminic activities and central nervous controlling activities.

It is reported that aripiprazole (7-{4-[4-(2,3-dichlorophenyl)-1-piperazinyl]butoxy}-3,4-dihydro-carbostyril, also known as, OPC-14597, BMS-337,039 and OPS-31) binds with high affinity to dopamine D₂ receptors and with moderate affinity to dopamine D₃ and 5-HT₇ receptors (Masashi Sasa et al., CNS Drug Reviews, Vol. 3, No. 1, pp. 24-33).

Further, it is reported that aripiprazole possesses presynaptic dopaminergic autoreceptor agonistic activity, postsynaptic D₂ receptor antagonistic activity, and D₂ receptor partial agonistic activity (T. Kikuchi, K. Tottori, Y. Uwahodo, T. Hirose, T. Miwa, Y. Oshiro and S. Morita: J. Pharmacol. Exp. Ther., Vol. 274, pp. 329, (1995); T. Inoue, M. Domae, K. Yamada and T. Furukawa: J. Pharmacol. Exp. Ther., Vol. 277, pp. 137, (1996)).

However, it has not been reported that compounds in the present invention have agonistic activity at 5-HT_{1A} receptor subtype.

It has been reported that therapeutic interventions using 5-HT_{1A} receptor ligands may be useful drug treatments for alcohol abuse (Mark Kleven et al., European Journal of Pharmacology, Vol. 281, (1995) pp. 219-228).

It is also reported that 5-HT_{1A} agonist drugs may be useful for the treatment and/or prophylaxis of disorders associated with neuronal degeneration resulting from ischemic events in mammals (U.S. Patent No. 5,162,375).

It is also reported that 5-HT_{1A} receptor hypersensitivity could be the biological basis for the increased frequency of migraine attack in stressful and anxious conditions (Massimo Leone et al., Neuro Report, Vol. 9, pp. 2605-2608(1998)).

It has recently been reported that (-)-(R)-2-[4-[[(3,4-dihydro-2H-1-benzopyran-2-yl)methyl]amino]-butyl]-1,2-benzisothiazol-3(2H)-one 1,1-dioxide monohydrochrolide (BAY-3702), a 5-HT_{1A} receptor agonist, has neuroprotective, anxiolytic- and antidepressant-like effects in animal models (Jean De Vry et al., European Journal of Pharmacology, Vol. 357, (1998), pp. 1-8).

It is also reported that 5-HT_{1A} receptor agonists appear to be broad spectrum antiemetic agents (Mary C. Wolff et al., European Journal of Pharmacology, Vol. 340, (1997), pp. 217-220; AB Alfieri et al., British Journal of Cancer, (1995), Vol. 72, pp. 1013-1015; Mary C. Wolff et al., Pharmacology Biochemistry and Behavior, 1995, Vol. 52, No. 3, pp. 571-575; James B. Lucot, European Journal of Pharmacology, 1997, Vol. 253, pp. 53-60).

Serotonin plays a role in several neurological and psychiatric disorders, including Alzheimer's disease, depression, nausea and vomiting, eating disorders, and migraine. (See Rasmussen et al., "Chapter 1. Recent Progress in Serotonin 5HT1A Receptor Modulators", in Annual Reports in Medicinal Chemistry, Vol. 30, Section I, pp. 1-9, 1995, Academic Press, Inc.). WO 00/16777 discloses that a 5HT_{1A} receptor agonist, buspirone is efficacious in treating a variety of symptoms associated with ADHD, and that combined use of a D2 receptor agonist and 5-HT_{1A} agonist provides effective treatments for ADHD and Parkinson's disease.

5HT_{1A} agonists are effective in the treatment of cognitive impairment in Alzheimer's disease, Parkinson's disease or senile dementia. US 5824680 discloses that a 5-HT_{1A} agonist, ipsapirone, is effective in treating Alzheimer's disease by improving memory. US 4687772 describes that a 5-HT_{1A} partial agonist, buspirone, is useful for improving short term memory in patients in need of treatment. WO 93/04681 discloses that use of 5-HT_{1A} partial agonists have been used for the treatment or prevention of cognitive disorders associated with Alzheimer's disease, Parkinson's disease or senile dementia.

5HT_{1A} agonists are also effective in the treatment of depression. US 4771053 describes that a 5-HT_{1A} receptor partial agonist, gepirone, is useful in alleviation of certain primary depressive disorders, such as severe depression, endogenous depression, major depression with melancholia, and atypical depression. WO 01/52855 discloses that the combined use of the 5-HT_{1A} receptor partial agonist gepirone with an antidepressant can effectively treat depression.

The 5-HT_{1A} receptor partial agonist buspirone alleviates motor disorders such as neuroleptic induced parkinsonism and extrapyramidal symptoms. These observations are disclosed in US 4438119. Furthermore 5-HT_{1A} agonists reverse neuroleptic-induced catalepsy in rodents, which mimic movement impairments observed in Parkinson's disease (Mark J. Millan, Journal of Pharmacology and Experimental Therapeutics, 2000, Vol. 295, p853-861). Thus, outside the scope of the invention aripiprazole can be used to manage psychosis in geriatric patients, Alzheimer's disease, Parkinson's disease or senile dementia, since it possesses potent, partial agonistic activities at D₂ and 5-HT_{1A} receptors. In addition, these patients might not experience extrapyramidal symptoms due to this property of aripiprazole.

Under these circumstances, the development of a safe antipsychotic drug with potent, full or partial agonist activity at 5-HT_{1A} receptors is earnestly desired.

The carbostyril compound in the present invention binds with high affinity and displays a potent, partial agonist activity at the 5-HT_{1A} receptors and it has higher intrinsic activity (about 68%) as compared with that of clozapine. Therefore, the compound in the present invention has a 5-HT_{1A} receptor agonistic activity that is more potent than the agonistic activity of clozapine.

P.E. Keck et al., Adv. in Pathophysiology and Treatment of Psych. Disorders, 85 (3), 645-661(2001) report on known medication for bipolar disorders and further list a variety of potential candidates for further investigation, including aripiprazole.

WO 03/026659, which represents prior art under Art. 54 (3) EPC, discloses a low hygroscopic aripiprazole formulation and its use for the treatment of, inter alia, mania including bipolar disorder acute mania and acute mania, bipolar disorder.

### SUMMARY OF THE INVENTION

It is an object of the present invention to provide a compound for use in treating a patient suffering from a disorder of the central nervous system associated with the 5-HT_{1A} receptor subtype, and more precisely bipolar disorders as defined in the claims.

### DETAILED DESCRIPTION OF THE INVENTION

To solve the above problem the present invention provides a compound, which is a carbostyril compound of the formula (1) : 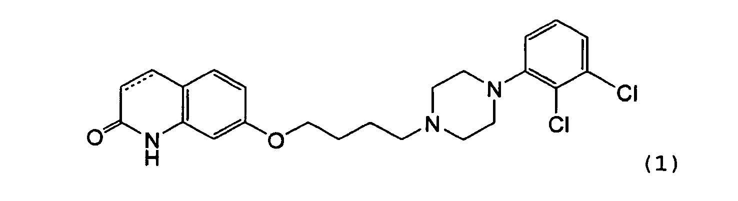 wherein the dotted line represents a single or a double bond, or a pharmaceutically acceptable salt or solvate thereof, for use in the treatment of disorders of the central nervous system associated with 5-HT_{1A} receptor subtype, selected from
(i) bipolar I disorder with most recent hypomanic, manic, mixed, depressed or unspecific episode, and
(ii) bipolar II disorder with recurrent major depressive episodes with hypomanic episodes, and cyclothymic disorder;
with the proviso that the following forms of aripiprazole are excluded:
(i) crystalline anhydrous form B having
   - characteristic peaks at 2θ in the powder X-ray diffraction spectrum at 11.0°, 16.6°, 19.3°, 20.3° and 22.1°,
   - characteristic absorption bands in the IR (KBr) spectrum at 2945, 2812, 1678, 1627, 1448, 1377, 1173, 960 and 779 cm⁻¹,
   - an endothermic peak near about 141.5°C in thermogravimetric/differential thermal analysis (heating rate 5°C/min),
   - an endothermic peak at about 140.7°C in differential scanning calorimetry (heating rate 5°C/min), and
   - a hygroscopicity corresponding to a moisture content of 0.40% or less after storage at 60°C and a humidity level of 100% for 24 h;
(ii) crystalline anhydrous form C having
   - characteristic peaks at 2θ in the powder X-ray diffraction spectrum at 12.6°, 13.7°, 15.4°, 18.1°, 19.0°, 20.6°, 23.5° and 26.4°,
   - characteristic absorption bands in the IR (KBr) spectrum at 2939, 2804, 1680, 1375 and 780 cm⁻¹,
   - an endothermic peak at about 150.2°C in thermogravimetric/differential thermal analysis (heating rate 5°C/min), and
   - characteristic peaks in the solid ¹³C-NMR spectrum at 32.8 ppm, 60.8 ppm, 74.9 ppm, 104.9 ppm, 152.2 ppm, 159.9 ppm and 175.2 ppm;
(iii) crystalline anhydrous form D having
   - characteristic peaks at 2θ in the powder X-ray diffraction spectrum at 8.7°, 11.6°, 16.3°, 17.7°, 18.6°, 20.3°, 23.4° and 25.0°,
   - characteristic absorption bands in the IR (KBr) spectrum at 2946, 1681, 1375, 1273, 1175 and 862 cm⁻¹,
   - endothermic peaks at about 136.8°C and 141.6°C in thermogravimetric/differential thermal analysis (heating rate 5°C/min), and
   - characteristic peaks in the solid ¹³C-NMR spectrum at 32.1 ppm, 62.2 ppm, 66.6 ppm, 104.1 ppm, 152.4 ppm, 158.4 ppm, and 174.1 ppm;
(iv) crystalline anhydrous form E having
   - characteristic peaks at 2θ in the powder X-ray diffraction spectrum at 8.0°, 13.7°, 14.6°, 17.6°, 22.5° and 24.0°,
   - characteristic absorption bands in the IR (KBr) spectrum at 2943, 2817, 1686, 1377, 1202, 969 and 774 cm⁻¹, and
   - an endothermic peak at about 146.5°C in thermogravimetric/differential thermal analysis (heating rate 5°C/min);
(v) crystalline anhydrous form F having
   - characteristic peaks at 2θ in the powder X-ray diffraction spectrum at 11.3°, 13.3°, 15.4°, 22.8°, 25.2° and 26.9°,
   - characteristic absorption bands in the IR (KBr) spectrum at 2940, 2815, 1679, 1383, 1273, 1177, 1035, 963 and 790 cm⁻¹, and
   - endothermic peaks at about 137.5°C and 149.8°C in thermogravimetric/differential thermal analysis (heating rate 5°C/min); and
(vi) crystalline anhydrous form G having
   - characteristic peaks at 2θ in the powder X-ray diffraction spectrum at 10.1°, 12.8°, 15.2°, 17.0°, 17.5°, 19.1°, 20.1°, 21.2°, 22.4°, 23.3°, 24.5° and 25.8°,
   - characteristic absorption bands in the IR (KBr) spectrum at 2942, 2813, 1670, 1625, 1377, 1195, 962 and 787 cm⁻¹, and
   - an endothermic peak at about 141.0°C and an exothermic peak at about 122.7°C in thermogravimetric/differential thermal analysis (heating rate 5°C/min).

The compounds of the forgoing general formula (1) are known compounds, which are disclosed in publication such as U.S. Pat. No. 5,006,528 or which can be readily prepared by the processes described in the above publication.

The carbostyril derivative represented by the formula (1) in the present invention can easily be converted into its acid-addition salt by reacting it with a pharmaceutically acceptable acid. Examples of such acid include inorganic acids, such as hydrochloric acid, sulfuric acid, phosphoric acid and hydrobromic acid; and organic acids, such as oxalic acid, maleic acid, fumaric acid, malic acid, tartaric acid, citric acid and benzoic acid.

The solvent of solvates is a solvent conventionally used in recrystallization. Examples of solvates include hemihydrates, hydrates, and alcoholates, such as ethanolates, methanolates and isopropanolates.

The desired compounds, prepared by the reactions mentioned above, can easily be isolated and purified by usual separation procedures such as solvent extraction, dilution, recrystallization, column chromatography and preparative thin layer chromatography.

The potent, partial 5-HT_{1A} receptor agonist in the present invention is useful for various disorders of the central nervous system associated with the 5-HT_{1A} receptor subtype that induces bipolar disorders, such as bipolar I disorder with most recent hypomanic, manic, mixed, depressed or unspecified episode; bipolar II disorder with recurrent major depressive episodes with hypomanic episodes, and cyclothymic disorder.

The Compounds for use of the present invention may be suitably prepared into pharmaceutically acceptable formulations (see U.S. Patent No. 5,006,528, European Patent No. 367,141 and Japanese Kokai (Laid-open) 7-304,740 (1995), and Japanese Patent Application No. 2000-194976 ).

The dosage of these pharmaceutical preparations of the invention may be selected appropriately depending on the method of administration, the patient's age, sex and other factors, severity of the disease and other factors. Generally, however, the daily dose of the active ingredient compound is preferably within the range of about 0.0001 to about 50 mg per kilogram of body weight. It is desirable that the active ingredient compound be contained in each unit dosage form in an amount of about 0.001 to about 1,000 mg, particularly 0.01 to 100 mg, more particularly 0.1 to 50 mg, yet more particularly 1 mg to 20 mg.

### Pharmacological tests

### 1. MATERIALS AND METHODS

### 1.1 Test Compound

7-{4-[4-(2,3-Dichlorophenyl)-1-piperazinyl]-butoxy}-3,4-dihydrocarbostyril (aripiprazole) was used as test compound.

### 1.2 Reference Compounds

Serotonin (5-HT) and WAY-100635 (N-[2-[4-(2-methoxyphenyl)-1-piperazinyl]ethyl]-N-(2-pyridimyl)-cyclohexanecarboxamide, a 5-HT_{1A} receptor antagonist, manufactured by RBI (Natick, MA) were used as reference compounds.

### 1.3 Vehicle

Dimethyl sulfoxide (DMSO) manufactured by Sigma Chemical Co. (St. Louis, MO) was used as vehicle.

### 1.4 Preparation of Test and Reference Compounds

Test compound was dissolved in 100% dimethyl sulfoxide (DMSO) to yield 100 µM stock solutions (final concentration of DMSO in all tubes containing test compound was 1%, v/v). All other reference compounds were prepared by the same method using double-distilled water rather than DMSO.

### 1.5 Experimental Procedure for the [³⁵S]GTP_{γ}S Binding Assay

Test and reference compounds were studied in triplicate at 10 different concentrations (0.01, 0.1, 1, 5, 10, 50, 100, 1000, 10000 and 50000 nM) for their effects upon basal [³⁵S]GTP_{γ}S binding to h5-HT_{1A} CHO cell membranes. Reactions were performed in 5 ml glass test tubes containing 8 µl of test/reference drug mixed with 792 µl of buffer (25 mM Tris HCl, 50 mM NaCl, 5 mM MgCl₂, 0.1 mM EGTA, pH = 7.4) containing GDP (1 µM), [³⁵S]GTP_{γ}S (0.1 nM) and h5-HT_{1A} CHO cell membranes (10 µg protein/reaction; NEN Life Science Products, Boston, MA; catalog # CRM035, lot # 501-60024, GenBank # X13556). Reactions proceeded for 60 min at room temperature and were terminated by rapid filtration through Whatman GF/B filter paper, using a Brandel harvester and 4x3 ml ice-cold buffer washes. ³⁵S radioactivity bound to the filter paper was measured using liquid scintillation counting (1272 Clinigamma, LKB/Wallach).

### 1.6 Experimental Procedure to Determine the Binding Affinity of Test compound (aripiprazole) at the h5-HT_{1A} Receptor

Test compound was studied in triplicate at 10 different concentrations (0.01, 0.1, 1, 10, 50, 100, 500, 1000, 5000 and 10000 nM) to determine its displacement of [³H]8-OH-DPAT (1 nM; NEN Life Sciences; catalog # NET 929, lot # 3406035, Specific Activity = 124.9 Ci/mmol) binding to h5-HT_{1A} receptors in CHO cell membranes (15 - 20 µg protein; NEN Life Science Products, catalog # CRM035, lot # 501-60024). Membranes (396 µl) were incubated in 5 ml glass tubes containing [³H]8-OH-DPAT (396 µl), test compound or vehicle (8 µl) and buffer A (50 mM Tris.HCl, 10 mM MgSO₄, 0.5 mM EDTA, 0.1% (w/v) ascorbic acid, pH = 7.4). All assays proceeded for 60 min at room temperature and were terminated by rapid filtration through Whatman GF/B filter paper (presoaked in buffer B; 50 mM Tris.HCl, pH = 7.4), using a Brandel harvester and 4x1 ml ice-cold washes with buffer B. Non-specific binding was determined in the presence of 10 µM (+)8-OH-DPAT.

### 1.7 Parameters Determined

Serotonin (5-HT) is a full 5-HT_{1A} receptor agonist which stimulates increases in basal [³⁵S]GTP_{γ}S binding to h5-HT_{1A} receptors in recombinant CHO cell membranes. Test compound was studied at 10 concentrations to determine their effects upon basal [^{3S}S] GTP_{γ}S binding relative to that produced by 10 µM 5-HT. The relative potency (EC₅₀, 95% confidence interval) and intrinsic agonist activity (% of Eₘₐₓ for 10 µM 5-HT) was calculated for each compound by computerized non-linear regression analysis of complete concentration-effect data. The binding affinity of test compound at the h5-HT_{1A} receptor was determined by its ability to prevent [³H]8-OH-DPAT binding to CHO cell membranes that express this receptor. Non-linear regression analysis of the competition binding data was used to calculate an inhibition constant (IC₅₀, 95% confidence interval), which is the concentration of test compound that occupies half of the h5-HT_{1A} sites specifically bound by [³H]8-OH-DPAT. The affinity of h5-HT_{1A} receptors for test compound (Ki, 95% confidence interval) was calculated by the equation, Ki = (IC₅₀) / (1+([[³H]8-OH-DPAT]/Kd), where the Kd for [³H]8-OH-DPAT at h5-HT_{1A} = 0.69 nM (NEN Life Sciences). All estimates of drug binding affinity, potency and intrinsic efficacy at the h5-HT_{1A} receptor were calculated using GraphPad Prism version 3.00 for Windows (GraphPad Software, San Diego, CA).

### 2. RESULTS

Test compound and 5-HT produced concentration-dependent increases above basal [³⁵S]GTP_{γ}S binding. 1% DMSO tested alone had no effect upon basal or drug-induced [³⁵S]GTP_{γ}S binding.

Test compound (EC₅₀ = 2.12 nM), 5-HT (EC₅₀ = 3.67 nM), potently stimulated basal [³⁵S]GTP_{γ}S binding. Potency and intrinsic agonist efficacy estimates were derived by non-linear regression analysis with correlation coefficients (r²)>0.98 in each case (Table 1). Test compound exerted partial agonist efficacies in the 65 - 70% range. WAY-100635 produced no significant change (unpaired Student's t-test) in basal [³⁵S]GTP_{γ}S binding at all concentrations tested (Table 1). WAY-100635 did, however, completely inhibit the effects of 5-HT and test compound upon [^{3S}S]GTP_{γ}S binding to h5-HT_{1A} receptors in CHO cell membranes (Table 2). Tables 1 and 2 are shown below.

Test compound demonstrated high affinity binding to h5-HT_{1A} receptors in CHO cell membranes (IC₅₀ = 4.03 nM, 95% confidence interval = 2.67 to 6.08 nM; Ki = 1.65 nM, 95% confidence interval = 1.09 to 2.48 nM).

**Table 1 Potency (EC₅₀) and Intrinsic Agonist Efficacy (Eₘₐₓ) of Test compound and Reference Drugs in a h5-HT_{1A} [³⁵S]GTP_{γ}S CHO-cell Membrane Binding Assay.**

| Drug | EC₅₀, nM (95% Confidence Interval) | Eₘₐₓ (% ± SEM) | Goodness of Fit (r²) |
|---|---|---|---|
| Test Compound | 2.12 (0.87 to 5.16) | 68.13 ± 3.16 | 0.986 |
| 5-HT | 3.67 (1.56 to 8.63) | 98.35 ± 4.47 | 0.986 |
| WAY-100635 | - | - | - |

**Table 2 Inhibitory Potency (IC₅₀) of WAY-100635 versus 1 µM Concentration of 5-HT and Test compound in a h5-HT_{1A} [³⁵S] GTP_{γ}S CHO-cell Membrane Binding Assay.**

| Drug Combination | WAY-100635 Inhibition Potency, IC₅₀, nM (95% Confidence Interval) | Goodness of Fit (r²) |
|---|---|---|
| 5-HT + WAY-100635 | 217.1 (127.4 to 369.7) | 0.988 |
| Test compound + WAY-100635 | 392.2 (224.1 to 686.2) | 0.989 |

## Claims

1. A compound, which is a carbostyril compound of the formula (1) : 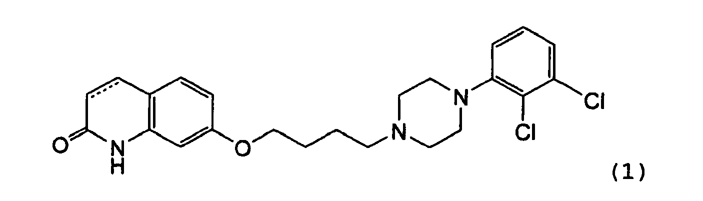 wherein the dotted line represents a single or a double bond, or a pharmaceutically acceptable salt or solvate thereof, for use in the treatment of disorders of the central nervous system associated with 5-HT_{1A} receptor subtype, selected from
(i) bipolar I disorder with most recent hypomanic, manic, mixed, depressed or unspecific episode, and
(ii) bipolar II disorder with recurrent major depressive episodes with hypomanic episodes, and cyclothymic disorder;
with the proviso that the following forms of aripiprazole are excluded:
(i) crystalline anhydrous form B having
- characteristic peaks at 2θ in the powder X-ray diffraction spectrum at 11.0°, 16.6°, 19.3°, 20.3° and 22.1°,
- characteristic absorption bands in the IR (KBr) spectrum at 2945, 2812, 1678, 1627, 1448, 1377, 1173, 960 and 779 cm⁻¹,
- an endothermic peak near about 141.5°C in thermogravimetric/differential thermal analysis (heating rate 5°C/min),
- an endothermic peak at about 140.7°C in differential scanning calorimetry (heating rate 5°C/min), and
- a hygroscopicity corresponding to a moisture content of 0.40% or less after storage at 60°C and a humidity level of 100% for 24 h;
(ii) crystalline anhydrous form C having
- characteristic peaks at 2θ in the powder X-ray diffraction spectrum at 12.6°, 13.7°, 15.4°, 18.1°, 19.0°, 20.6°, 23.5° and 26.4°,
- characteristic absorption bands in the IR (KBr) spectrum at 2939, 2804, 1680, 1375 and 780 cm⁻¹,
- an endothermic peak at about 150.2°C in thermogravimetric/differential thermal analysis (heating rate 5°C/min), and
- characteristic peaks in the solid ¹³C-NMR spectrum at 32.8 ppm, 60.8 ppm, 74.9 ppm, 104.9 ppm, 152.2 ppm, 159.9 ppm and 175.2 ppm;
(iii) crystalline anhydrous form D having
- characteristic peaks at 2θ in the powder X-ray diffraction spectrum at 8.7°, 11.6°, 16.3°, 17.7°, 18.6°, 20.3°, 23.4° and 25.0°,
- characteristic absorption bands in the IR (KBr) spectrum at 2946, 1681, 1375, 1273, 1175 and 862 cm⁻¹,
- endothermic peaks at about 136.8°C and 141.6°C in thermogravimetric/differential thermal analysis (heating rate 5°C/min), and
- characteristic peaks in the solid ¹³C-NMR spectrum at 32.1 ppm, 62.2 ppm, 66.6 ppm, 104.1 ppm, 152.4 ppm, 158.4 ppm, and 174.1 ppm;
(iv) crystalline anhydrous form E having
- characteristic peaks at 2θ in the powder X-ray diffraction spectrum at 8.0°, 13.7°, 14.6°, 17.6°, 22.5° and 24.0°,
- characteristic absorption bands in the IR (KBr) spectrum at 2943, 2817, 1686, 1377, 1202, 969 and 774 cm⁻¹, and
- an endothermic peak at about 146.5°C in thermogravimetric/differential thermal analysis (heating rate 5°C/min) ;
(v) crystalline anhydrous form F having
- characteristic peaks at 2θ in the powder X-ray diffraction spectrum at 11.3°, 13.3°, 15.4°, 22.8°, 25.2° and 26.9°,
- characteristic absorption bands in the IR (KBr) spectrum at 2940, 2815, 1679, 1383, 1273, 1177, 1035, 963 and 790 cm⁻¹, and
- endothermic peaks at about 137.5°C and 149.8°C in thermogravimetric/differential thermal analysis (heating rate 5°C/min) ; and
(vi) crystalline anhydrous form G having
- characteristic peaks at 2θ in the powder X-ray diffraction spectrum at 10.1°, 12.8°, 15.2°, 17.0°, 17.5°, 19.1°, 20.1°, 21.2°, 22.4°, 23.3°, 24.5° and 25.8°,
- characteristic absorption bands in the IR (KBr) spectrum at 2942, 2813, 1670, 1625, 1377, 1195, 962 and 787 cm⁻¹, and
- an endothermic peak at about 141.0°C and an exothermic peak at about 122.7°C in thermogravimetric/differential thermal analysis (heating rate 5°C/min).

2. The compound for use of claim 1, wherein the disorder is bipolar I disorder.

3. The compound for use of claim 1, wherein the disorder is bipolar II disorder.

4. The compound for use of any of claims 1-3, wherein the carbostyril compound is 7-{4-[4-(2,3-dichlorophenyl)-1-piperaziny]butoxyl}-3,4-dihydrocarbostyril.

## Patentansprüche

1. Verbindung, welche eine Carbostyrilverbindung der Formel (1) ist: 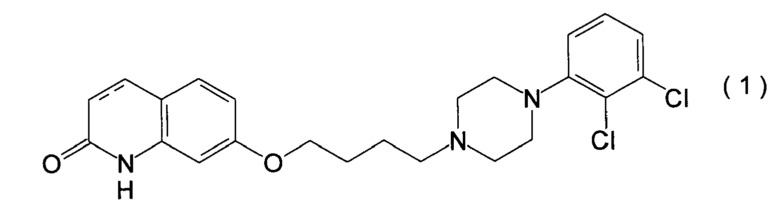 worin die gestrichelte Linie eine Einfach- oder Doppelbindung darstellt, oder ein pharmazeutisch akzeptables Salz oder Solvat davon, zur Anwendung bei der Behandlung von Störungen des zentralen Nervensystems, die mit dem 5-HT_{1A}-Rezeptor-Subtyp im Zusammenhang stehen, ausgewählt aus
(i) einer bipolaren Störung vom Typ I mit einer zuletzt hypomanischen, manischen, gemischten, deprimierten oder unspezifischen Episode, und
(ii) einer bipolaren Störung vom Typ II mit wiederkehrenden grossen depressiven Episoden mit hypomanischen Episoden und cyclothymischer Störung;
mit der Bedingung, dass die folgenden Formen von Aripiprazol ausgeschlossen sind:
(i) eine kristalline wasserfreie Form B mit
- charakteristischen Peaks bei 2 θ im Pulverröntgendiffraktometriespektrum bei 11,0°, 16,6°, 19,3°, 20,3° und 22,1°,
- charakteristischen Absorptionsbanden im IR (KBr)-Spektrum bei 2.945, 2.812, 1.678, 1.627, 1.448, 1.377, 1.173, 960 und 779 cm⁻¹,
- einem endothermen Peak in der Nähe von etwa 141,5°C in der thermogravimetrischen Differential-Thermoanalyse (Heizrate: 5°C/min),
- einem endothermen Peak bei etwa 140,7°C in der Differentialrasterkalorimetrie (Heizrate: 5°C/min), und
- einer Hygroskopizität, entsprechend einem Feuchtigkeitsgehalt von 0,40 % oder weniger nach Lagerung bei 60°C und einem Feuchtigkeitsgrad von 100 % für 24 Stunden;
(ii) eine kristalline wasserfreie Form C mit
- charakteristischen Peaks bei 2 θ im Pulverröntgendiffraktometriespektrum bei 12,6°, 13,7°, 15,4°, 18,1°, 19,0°, 20,6°, 23,5° und 26,4°,
- charakteristischen Absorptionsbanden im IR (KBr)-Spektrum bei 2.939, 2.804, 1.680, 1.375 und 780 cm⁻¹,
- einem endothermen Peak bei etwa 150,2°C in der thermogravimetrischen Differential-Thermoanalyse (Heizrate: 5°C/min), und
- charakteristischen Peaks im Feststoff ¹³C-NMR-Spektrum bei 32,8 ppm, 60,8 ppm, 74,9 ppm, 104,9 ppm, 152,2 ppm, 159,9 ppm und 175,2 ppm;
(iii) eine kristalline wasserfreie Form D mit
- charakteristischen Peaks bei 2 θ im Pulverröntgendiffraktometriespektrum bei 8,7°, 11, 6°, 16, 3°, 17,7°, 18, 6°, 20,3°, 23,4° und 25,0°,
- charakteristischen Absorptionsbanden im IR (KBr)-Spektrum bei 2.946, 1.681, 1.375, 1.273, 1.175 und 862 cm⁻¹,
- endothermen Peaks bei etwa 136,8°C und 141,6°C in der thermogravimetrischen Differential-Thermoanalyse (Heizrate: 5°C/min), und
- charakteristischen Peaks im Feststoff-¹³C-NMR-Spektrum bei 32,1 ppm, 62,2 ppm, 66,6 ppm, 104,1 ppm, 152,4 ppm, 158,4 ppm und 174,1 ppm;
(iv) eine kristalline wasserfreie Form E mit
- charakteristischen Peaks bei 2 θ im Pulverröntgendiffraktometriespektrum bei 8,0°, 13,7°, 14,6°, 17,6°, 22,5° und 24,0°,
- charakteristischen Absorptionsbanden im IR (KBr)-Spektrum bei 2.943, 2.817, 1.686, 1.377, 1.202, 969 und 774 cm⁻¹, und
- einem endothermen Peak bei etwa 146,5°C in der thermogravimetrischen Differential-Thermoanalyse (Heizrate: 5°C/min);
(v) eine kristalline wasserfreie Form F mit
- charakteristischen Peaks bei 2 θ im Pulverröntgendiffraktometriespektrum bei 11,3°, 13,3°, 15,4°, 22,8°, 25,2° und 26, 9°,
- charakteristischen Absorptionsbanden im IR (KBr)-Spektrum bei 2.940, 2.815, 1.679, 1.383, 1.273, 1.177, 1.035, 963 und 790 cm⁻¹, und
- endothermen Peaks bei etwa 137,5°C und 149,8°C in der thermogravimetrischen Differential-Thermoanalyse (Heizrate: 5°C/min); und
(vi) eine kristalline wasserfreie Form G mit
- charakteristischen Peaks bei 2 θ im Pulverröntgendiffraktometriespektrum bei 10, 1°, 12,8°, 15,2°, 17,0°, 17,5°, 19,1°, 20,1°, 21,2°, 22,4°, 23,3°, 24,5° und 25,8°,
- charakteristischen Absorptionsbanden im IR (KBr)-Spektrum bei 2.942, 2.813, 1.670, 1.625, 1.377, 1.195, 962 und 787 cm⁻¹, und
- einem endothermen Peak bei etwa 141,0°C und einen exothermen Peak bei etwa 122,7°C in der thermogravimetrischen Differential-Thermoanalyse (Heizrate: 5°C/min).

2. Verbindung zur Verwendung gemäss Anspruch 1, wobei die Störung eine bipolare Störung vom Typ I ist.

3. Verbindung zur Verwendung gemäss Anspruch 1, wobei die Störung eine bipolare Störung vom Typ II ist.

4. Verbindung zur Verwendung gemäss einem der Ansprüche 1 bis 3, wobei die Carbostyrilverbindung 7-{4-[4-(2,3-Dichlorphenyl)-1-piperazinyl]butoxyl}-3,4-dihydrocarbostyril ist.

## Revendications

1. Composé qui est un composé carbostyryle de formule (1) : 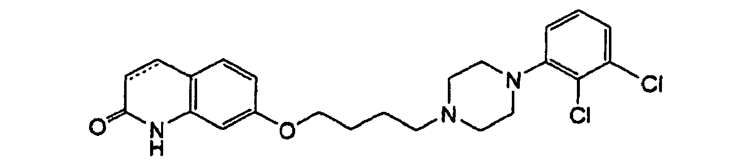 dans laquelle la ligne de pointillé représente une liaison simple ou double, ou un sel ou solvate pharmaceutiquement acceptable de celui-ci, pour une utilisation dans le traitement de troubles du système nerveux central associés à un sous-type de récepteur de 5-HT_{1A} et choisis parmi :
(i) les troubles bipolaires de type I avec épisode hypomaniaque, maniaque, mixte, dépressif ou non spécifique très récent, et
(ii) les troubles bipolaires de type II avec des épisodes dépressifs majeurs récurrents associés à des épisodes hypomaniaques, et des troubles cyclothymiques ;
à l'exclusion des formes suivantes d'aripiprazole :
(i) la forme anhydre cristalline B ayant
- des pics caractéristiques à 2θ dans le spectre de diffraction des rayons X par la technique des poudres à 11,0°, 16,6°, 19,3°, 20,3° et 22,1°,
- des bandes d'absorption caractéristiques dans le spectre IR (KBr) à 2945, 2812, 1678, 1627, 1448, 1377, 1173, 960 et 779 cm⁻¹,
- un pic endothermique proche d'environ 141,5°C par analyse thermogravimétrique/thermique différentielle (vitesse de montée en température 5°C/min),
- un pic endothermique à environ 140,7°C par calorimétrie à balayage différentiel (vitesse de montée en température 5°C/min), et
- une hygroscopicité correspondant à une teneur en humidité de 0,40 % ou moins après stockage à 60°C et à un taux d'humidité de 100 % pendant 24 heures ;
(ii) la forme anhydre cristalline C ayant
- des pics caractéristiques à 2θ dans le spectre de diffraction des rayons X par la technique des poudres à 12,6°, 13,7°, 15,4°, 18,1°, 19,0°, 20,6°, 23,5° et 26,4°,
- des bandes d'absorption caractéristiques dans le spectre IR (KBr) à 2939, 2804, 1680, 1375 et 780 cm⁻¹,
- un pic endothermique à environ 150,2°C par analyse thermogravimétrique/thermique différentielle (vitesse de montée en température 5°C/min), et
- des pics caractéristiques dans le spectre de RMN⁻¹³C solide à 32,8 ppm, 60,8 ppm, 74,9 ppm, 104,9 ppm, 152,2 ppm, 159,9 ppm et 175,2 ppm ;
(iii) la forme anhydre cristalline D ayant
- des pics caractéristiques à 2θ dans le spectre de diffraction des rayons X par la technique des poudres à 8,7°, 11,6°, 16,3°, 17,7°, 18,6°, 20,3°, 23,4° et 25,0°,
- des bandes d'absorption caractéristiques dans le spectre IR (KBr) à 2946, 1681, 1375, 1273, 1175 et 862 cm⁻¹,
- des pics endothermiques à environ 136,8°C et 141,6°C par analyse thermogravimétrique/ thermique différentielle (vitesse de montée en température 5°C/min), et
- des pics caractéristiques dans le spectre de RMN⁻¹³C solide à 32,1 ppm, 62,2 ppm, 66,6 ppm, 104,1 ppm, 152,4 ppm, 158,4 ppm et 174,1 ppm ;
(iv) la forme anhydre cristalline E ayant
- des pics caractéristiques à 2θ dans le spectre de diffraction des rayons X par la technique des poudres à 8,0°, 13,7°, 14,6°, 17,6°, 22,5° et 24,0°,
- des bandes d'absorption caractéristiques dans le spectre IR (KBr) à 2943, 2817, 1686, 1377, 1202, 969 et 774 cm⁻¹, et
- un pic endothermique à environ 146,5°C par analyse thermogravimétrique/thermique différentielle (vitesse de montée en température 5°C/min) ;
(v) la forme anhydre cristalline F ayant
- des pics caractéristiques à 2θ dans le spectre de diffraction des rayons X par la technique des poudres à 11,3°, 13,3°, 15,4°, 22,8°, 25,2° et 26,9°,
- des bandes d'absorption caractéristiques dans le spectre IR (KBr) à 2940, 2815, 1679, 1383, 1273, 1177, 1035, 963 et 790 cm⁻¹, et
- des pics endothermiques à environ 137,5°C et 149,8°C par analyse thermogravimétrique/ thermique différentielle (vitesse de montée en température 5°C/min) ; et
(vi) la forme anhydre cristalline G ayant
- des pics caractéristiques à 2θ dans le spectre de diffraction des rayons X par la technique des poudres à 10,1°, 12,8°, 15,2°, 17,0°, 17,5°, 19,1°, 20,1°, 21,2°, 22,4°, 23,3°, 24,5° et 25,8°,
- des bandes d'absorption caractéristiques dans le spectre IR (KBr) à 2942, 2813, 1670, 1625, 1377, 1195, 962 et 787 cm⁻¹, et
- un pic endothermique à environ 141,0°C et un pic exothermique à environ 122,7°C par analyse thermogravimétrique/thermique différentielle (vitesse de montée en température 5°C/min).

2. Composé pour utilisation selon la revendication 1, dans lequel le trouble est un trouble bipolaire de type 1.

3. Composé pour utilisation selon la revendication 1, dans lequel le trouble est un trouble bipolaire de type II.

4. Composé pour utilisation selon l'une quelconque des revendications 1 à 3, dans lequel le composé carbostyryle est le 7-{4-[4-(2,3-dichlorophényl)-1-pipérazinyl]butoxy}-3,4-dihydrocarbostyryle.
